# EUROPEAN PATENT SPECIFICATION

(11) **EP 2 945 582 B1**
(45) Date of publication and mention of the grant of the patent: **15.11.2017**
(21) Application number: 14700354.5
(22) Date of filing: 10.01.2014
(51) Int. Cl.: A61F 9/04

(54) **PROTECTIVE MASK**
SCHUTZMASKE
MASQUE DE PROTECTION

(30) Priority: 15.01.2013 IT PD20130001 U
(43) Date of publication of application: 25.11.2015
(73) Proprietor: Sorgato, Luca, 35020 Sant'Angelo di Piove di Sacco (PD) (IT)
(72) Inventor: Sorgato, Luca, 35020 Sant'Angelo di Piove di Sacco (PD) (IT)
(74) Representative: Cantaluppi, Stefano
(86) International application number: PCT/EP2014/050404
(87) International publication number: WO 2014/111317

(56) References cited:
- WO-A2-02/06881
- CN-Y- 201 005 105
- DE-A1- 3 422 065
- GB-A- 717 256
- US-A- 4 779 291
- US-A- 4 790 031
- US-A- 5 598 230
- US-A1- 2012 222 185

## Description

The invention concerns a transparent protective mask, principally designed to protect the user's eyes during the conduct of potentially dangerous activities such as, in particular, pyrotechnic activities. The mask also lends itself to other uses, for example in healthcare and hospital environments.

In this specific field, the dangers inherent in the handling and use of pyrotechnic items, better known as fireworks, are well known. These items cause many injuries to users every year, some of them extremely serious. Typical injuries are those caused to the limbs and face of persons who set off such fireworks without sufficient care and/or experience. Even more serious are the effects that they can have on young people and children if not assisted by responsible and adequately protected adults. Furthermore, there can be no disputing the fascination that the materials in question exert on those who are most exposed to them, who often experiment with pyrotechnics by setting off fireworks without the necessary protective measures.

Frequently, the failure to use adequate protective measures is attributable to the fact that such measures are not available in the place and at the time at which the decision is made to set off the fireworks. This often takes place at the homes of friends, where the proper protective measures are unavailable or distant from the place where the fireworks are played with.

Examples of protective masks are described in US4790031 A, US 4 779 291 A and CN 201 005 105 Y.

The principal purpose of the invention is to provide a protective mask specifically indicated for the safe use of pyrotechnic items.

This purpose is reflected in the provision of a mask structurally and functionally designed to be capable of being made available together with the pyrotechnic items so as to ensure their availability in the place and at the time at which it is decided to use them. For example, it is envisaged by the invention that the mask thus designed, which by its nature is easily packaged, can be coupled (for example, inserted) with the package that contains the pyrotechnic items.

The characteristics and benefits of the invention will be better appreciated from the detailed description of a preferred embodiment illustrated, for purely indicative and non-limitative purposes, in the attached drawings, where:
Figure 1 is a perspective view of a protective mask according to the invention in its fastened working state;
Figure 2 is a perspective view of the mask in Figure 1 in its unfastened state;

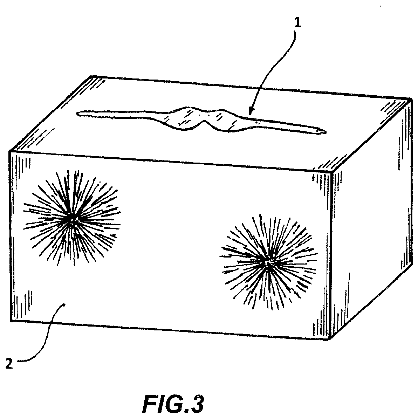

Figure 3 is a perspective view of the mask of the preceding figures coupled with a fireworks package.

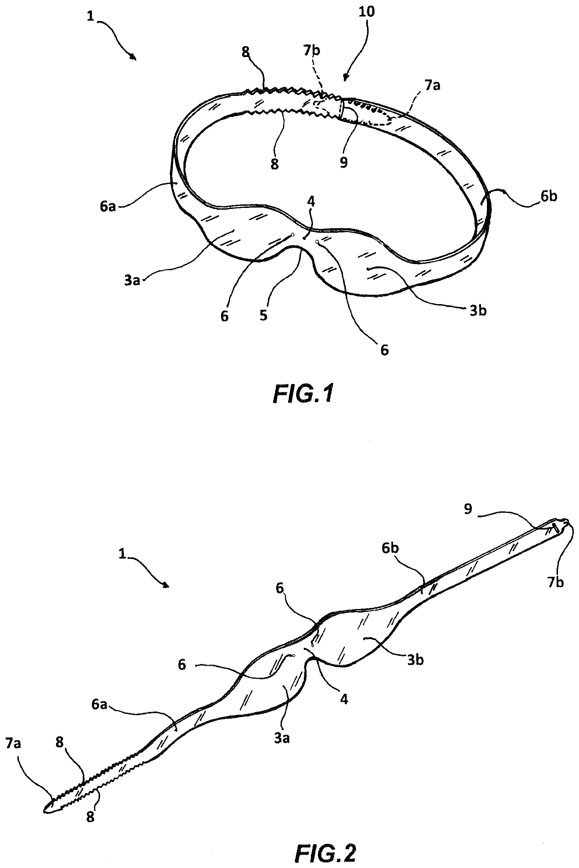

In the figures, 1 indicates the whole of a protective mask produced according to the invention, intended to be folded and prepared as illustrated in Figure 2 for subsequent coupling with a firework package 2. The mask 1 is cut by punching from a flexible sheet of transparent vinyl or polyester film, of the type used for example to create transparent window panels in fabric covers and in sails. It is also envisaged that the mask may be cut from sheets of soft, flexible and transparent PVC.

The mask 1 comprises, in a single piece, a pair of eye protectors in the form of lenses 3a,b interconnected by a bridge 4 that forms a hollow 5 to accommodate the user's nose. In the vicinity of the bridge 4, a small ventilation hole 6 is made for each lens in order to prevent any misting of the corresponding lens.

From each lens 3a,b a thin band 6a,b extends opposite the bridge 4 and intended to encircle the user's head and secure the mask 1 in the position of use.

The band 6a has a pointed free extremity 7a and, behind this, a plurality of teeth 8 on opposing edges. The band 6b also has a pointed free extremity 7b and, behind this a transverse incision 9 capable of receiving a length of the band 6a provided with the teeth 8 in order to attach the two bands 6a,b to one another and thus close the mask 1 in the fastened position on the user's head.

In other words, means of fastening 10 are provided on the two bands 6a,b in order to secure them to one another in a removable manner, and in the version of the mask 1 illustrated in the figures, these means of fastening 10 are formed by the connection between the edges provided with teeth 8 of the band 6a and the transverse incision 9 of the band 6b.

As an alternative to the means of fastening 10, it is possible to use other means of fastening that range from a simple knotting between the two bands 6a,b to mechanical means of connection, which may be adjustable. Once punched, the mask 1 may be inserted in the package 2 of fireworks in order to be sold to the user together with the fireworks. In this way, the user will be certain of having, when the fireworks are set off, at least the most important protection for preventing potential injuries, namely the protection that safeguards the integrity of the eyes. Since it is made from soft and flexible material, the mask 1 may be associated with the package 2 in any manner and without the need for any prior pre-packaging. For example, it may be simply inserted between the box of the package 2 and any protective film wrapped around the box, in the case of rectangular packages, or curled around the wall of the package in the case of cylindrical or more generally curved packages.

The mask 1, although designed for this specific use, also lends itself to other uses where it is necessary to have a form of protection for the face (or at least of the eyes) that can be kept in a small space, such as a pocket, a wallet or a handbag, without suffering any damage.

It is pointed out that although, in the version of the mask 1 illustrated in the figures, the means of fastening 10 are provided on the bands 6a,b, these could also be situated elsewhere, for example on the bridge 4.

The main benefit of the invention undoubtedly lies in the fact that it provides a direct coupling between pyrotechnic items and an associated form of protection, so as to ensure the availability of the said form of protection at the moment when the pyrotechnic items are set off and the user is most exposed to potential danger. Other benefits are the low cost of the protection, which can be produced as a disposable single-use item, its effectiveness due to the fact that it adjusts completely to the user's face, and its ease of use and stability once put on, thanks to the particular means of fastening that it uses.

## Claims

1. Transparent protective mask comprising:
• a pair of eye protectors in the form of lenses (3a,b),
• a bridge (4) interconnecting said eye protectors,
• a band (6a,b) extended from each lens (3a,b), opposite the bridge (4), and intended to encircle the user's head and secure the mask (1) in the position of use,
**characterised by** said band (6a,b), lenses (3a,b) and bridge (4) being transparent and formed as a single piece cut from a transparent flexible sheet.

2. Mask according to claim 1 comprising means of fastening (10) on the said bands (6a,b) in order to secure the said bands (6a,b) to one another in a removable manner.

3. Mask according to claim 2 in which the said means of fastening (10) comprise a toothed section (8) on the edges of one of the said bands (6a,b) and a transverse incision (9) on the other of the said bands (6a,b) capable of receiving the toothed section (8) of the other band in order to lock the two bands (6a,b) to one another and thus close the mask (1) in its fastened position on the user's head.

4. Mask according to claim 1 in which the material of the said sheet is a vinyl or polyester resin or PVC.

5. Mask according to one or more of the preceding claims including means of ventilation associated with the said eye protectors.

6. Mask according to claim 5 in which the said means of ventilation consist of holes made in the eye protectors in the vicinity of or adjacent to the said bridge.

7. Firework package (2) including at least one mask (1) according to one or more of the preceding claims.

## Patentansprüche

1. Transparente Schutzmaske umfassend:
- ein Paar Augen-Schützer in Form von Linsen (3a,b),
- eine Brücke (4), welche die Augen-Schützer verbindet,
- ein Band (6a,b), welches sich von jeder Linse (3a,b) erstreckt, gegenüber der Brücke (4) liegt und vorgesehen ist, um den Kopf des Anwenders zu umschließen und die Maske (1) in der Benutzungsposition zu sichern,
**dadurch gekennzeichnet, dass**
das Band (6a,b), die Linsen (3a,b) und die Brücke (4) transparent sind und als ein einziges Stück, welches aus einer transparenten, flexiblen Folie geschnitten ist, ausgebildet sind.

2. Maske gemäß Anspruch 1, umfassend Befestigungsmittel (10) an den Bändern (6a,b), um die Bänder (6a,b) miteinander auf lösbare Art und Weise zu sichern.

3. Maske gemäß Anspruch 2, wobei die Befestigungsmittel (10) einen gezahnten Abschnitt (8) an den Rändern von einem der Bänder (6a,b) und einen schrägen Einschnitt (9) an dem anderen der Bänder (6a,b), welcher geeignet ist, um den gezahnten Abschnitt (8) des anderen Bands aufzunehmen, umfasst, um die beiden Bänder (6a,b) zueinander zu verriegeln und somit die Maske (1) in dessen am Kopf des Anwenders befestigter Position zu schließen.

4. Maske gemäß Anspruch 1, wobei das Material der Folie ein Vinyl- oder PolyesterHarz oder PVC ist.

5. Maske gemäß einem oder mehreren der vorhergehenden Ansprüche, aufweisend Ventilationsmittel, welche mit den Augen-Schützern in Verbindung gebracht sind.

6. Maske gemäß Anspruch 5, wobei die Ventilationsmittel aus Löchern bestehen, welche in den Augen-Schützern in der Nähe oder angrenzend an die Brücke (4) ausgebildet sind.

7. Feuerwerk-Verpackung (2), aufweisend wenigstens eine Maske (1) gemäß einem der vorhergehenden Ansprüche.

## Revendications

1. Masque de protection transparent comprenant :
une paire d'éléments de protection des yeux formant des parties recouvrant les yeux (3 a, b),
un pont (4) interconnectant lesdits éléments de protection des yeux,
une bande (6a, b) s'étendant à partir de chaque partie recouvrant les yeux (3a, b), à l'opposé du pont (4), et conçue pour entourer la tête de l'utilisateur et fixer le masque (1) en position d'utilisation,
**caractérisé en ce que** :
ladite bande (6a, b), lesdites parties recouvrant les yeux (3a, b) et ledit pont (4) sont transparents et formés en une seule pièce coupée dans une feuille souple transparente.

2. Masque selon la revendication 1, comprenant un moyen de fixation (10) sur lesdites bandes (6a, b) de manière à fixer lesdites bandes (6a, b) l'une à l'autre de manière amovible.

3. Masque selon la revendication 2, dans lequel ledit moyen de fixation (10) comprend une section dentée (8) sur les bords de l'une desdites bandes (6a, b) et une incision transversale (9) sur l'autre desdites bandes (6a, b) capable de recevoir la section dentée (8) de l'autre bande de manière à bloquer les deux bandes (6a, b) l'une à l'autre et à ainsi fermer le masque (1) dans sa position fixée sur la tête de l'utilisateur.

4. Masque selon la revendication 1, dans lequel le matériau de ladite feuille est une résine vinyle ou polyester ou du PVC.

5. Masque selon au moins l'une des revendications précédentes, comprenant un moyen de ventilation associé auxdits éléments de protection des yeux.

6. Masque selon la revendication 5, dans lequel ledit moyen de ventilation est constitué de trous formés dans les éléments de protection des yeux à proximité dudit, ou adjacent audit, pont.

7. Emballage de feu d'artifice (2) comprenant au moins un masque (1) selon au moins l'une des revendications précédentes.
